# EUROPÄISCHE PATENTSCHRIFT

(11) **EP 3 148 396 B1**
(45) Veröffentlichungstag und Bekanntmachung des Hinweises auf die Patenterteilung: **12.12.2018**
(21) Anmeldenummer: 16728348.0
(22) Anmeldetag: 13.06.2016
(51) Int. Cl.: A61B 1/00

(54) **ENDOSKOPVERPACKUNG MIT ZWEI SEPARATEN KAMMERN**
ENDOSCOPE PACKAGING WITH TWO SEPARATE CHAMBERS
CONDITIONNEMENT POUR ENDOSCOPE MUNI DE DEUX COMPARTIMENTS SÉPARÉS

(30) Priorität: 14.08.2015 DE 102015113430
(43) Veröffentlichungstag der Anmeldung: 05.04.2017
(73) Patentinhaber: invendo medical GmbH, 86438 Kissing (DE)
(72) Erfinder: CIFARELLI, John F., Oyster Bay, NY 11771 (US); HERCEGFI, Timo, 69221 Dossenheim (DE); FIEBER, Markus, Dr., 80636 München (DE); ABICHT, Felix, 82337 Walchstadt (DE); ILG, Dylan, 82266 Inning (DE); VON RUEPPRECHT, Oliver, 86343 Königsbrunn (DE)
(74) Vertreter: Winter, Brandl, Fürniss, Hübner, Röss, Kaiser, Polte - Partnerschaft mbB
(86) Internationale Anmeldenummer: PCT/EP2016/063492
(87) Internationale Veröffentlichungsnummer: WO 2017/028980

(56) Entgegenhaltungen:
- DE-A1-102013 004 168
- DE-U1- 29 608 617
- US-A- 3 123 211

## Beschreibung

Die vorliegende Erfindung betrifft eine Endoskopverpackung, insbesondere eine sogenannte Blister- oder Klarsichtverpackung zum sterilen Verpacken eines Endoskops oder eines Teils davon, mit einem einstückig ausgebildeten und vorzugsweise rechteckförmigen Aufnahmeteil zur Aufnahme eines Endoskops und zumindest einer mit dem Aufnahmeteil zumindest randseitig lösbar verbundenen Abdeckung, insbesondere einer vorzugsweise durchsichtigen Folie, zum sterilen Verschließen des Aufnahmeteils, wobei das Aufnahmeteil aufweist: eine erste Kammer zur Aufnahme eines ersten Endoskopabschnitts, insbesondere eines proximal angeordneten Handhabungsabschnitts oder eines mit einem solchen koppelbaren Anschlussabschnitts, der dafür bestimmt und ausgelegt ist, im operativen Einsatz nicht mit dem Patienten in Kontakt zu gelangen, und eine zweite Kammer zur Aufnahme eines zweiten Endoskopabschnitts, insbesondere eines distal angeordneten Penetrierabschnitts mit vorzugsweise einem Endoskopschlauch und einem Endoskopkopf, der dafür bestimmt und ausgelegt ist, im operativen Einsatz mit einem Patienten in, insbesondere invasiven, Kontakt zu kommen.

### Hintergrund der Erfindung

Endoskope sind medizinische Arbeitsgerätschaften, mit denen das Innere von in der Regel lebenden Organismen untersucht und/oder manipuliert werden kann. Ursprünglich für die humanmedizinische Diagnostik entwickelt, werden sie heute auch für minimal-invasive operative Eingriffe an Mensch und Tier eingesetzt. Zu Endoskopen zählen sowohl starre als auch flexible Endoskope und deren Unterarten.

Ein starres Endoskop leitet die Bildinformationen des zu untersuchenden Objekts bzw. Raums durch ein Linsensystem im Inneren des Endoskopschafts bzw. im distalen Endoskopkopf an ein proximal angeordnetes Okular weiter. Beispiel hierfür sind das Arthroskop und Zystoskop.

Das für die Untersuchung notwendige Licht wird entweder durch ein distales Leuchtmittel (z. B. LED) im Endoskopkopf bereitgestellt oder über einen an ein proximales Leuchtmittel angeschlossenen Lichtleiter, ebenfalls im Inneren des Schafts, durch Glasfaserbündel an die Spitze des Endoskops transportiert.

Bei einem flexiblen Endoskop werden Bild und Licht ebenfalls häufig über Glasfaserbündel übertragen. Beispiel sind das Gastroskop, Koloskop und Bronchoskop.

Neben Bild und Licht können über den Endoskopschaft in darin vorgesehenen Arbeitskanälen mikromechanische Geräte wie kleine Zangen oder Greifer in den Untersuchungsraum eingeführt werden, aber auch Behandlungs- oder Spülflüssigkeiten hingefördert und Patientengewebe und/oder Körperflüssigkeiten abtransportiert werden.

Endoskope haben somit einen flexiblen oder starren Endoskopschaft, an dessen distalen Ende bzw. Endoskopkopf eine Beleuchtung (Lichtaustritt eines Lichtleiterkabels oder ein Leuchtkörper) sowie eine Optik (Linsensystem und/oder lichtempfindlicher Chip), innerhalb des Endoskopschafts ein oder mehrere Arbeitskanäle und an dem proximalen Ende des Endoskopschafts ein Handhabungsabschnitt oder ein mit einem solchen koppelbaren Anschlussabschnitt vorgesehen ist, welcher über diverse Leitungen und Signalleitungen mit einer Betriebsmaschine des Endoskops verbindbar ist, über welche das Endoskop angesteuert, mit Strom versorgt und die Bilddaten ausgewertet und visualisiert werden können.

Vor einer Behandlung eines Patienten muss somit das Endoskop mit dem proximalen Ende des Endoskopschafts bzw. dem Anschluss- oder Handhabungsabschnitt mit den Ver- und Entsorgungsleitungen der Betriebsmaschine gekoppelt werden. Das Endoskop, d.h. der Endoskopschaft samt Endoskopkopf und der Handhabungs-, Anschluss- oder Kopplungsabschnitt werden in sterilisierter Form und verpackt ausgeliefert.

Aus dem Stand der Technik wie bespielsweise DE 689 09 755 T2 ist bekannt, dass das Endoskop in einer Sterilverpackung ausgeliefert wird, bei der das Endoskop in einem Aufnahmeteil aufgenommen und mittels einer Abdeckung oder (Tyvek®-)Folie steril verschlossen ist. Um das ausgelieferter Endoskop mit der Betriebsmaschine bzw. den Ver- und Entsorgungsschläuchen und -leitungen verbinden zu können, muss die Verpackung geöffnet werden und das gesamte Endoskop herausgenommen werden. Dabei kann es passieren, dass der für die Behandlung vorgesehene mediale Abschnitt (Endoskopschlauch- oder schaft) und der distale Abschnitt (Endoskopfkopf) des Endoskops versehentlich kontaminiert werden, so dass dieses Endoskop gegen ein neues ausgetauscht werden muss, da man ansonsten Gefahr läuft, den zu behandelnden Patienten zu infizieren.

Die DE 10 2013 004168 A1 offenbart beispielsweise eine Verpackung von sterilen Operationswerkzeugen, wobei die Verpackung zweiteilig ausgebildet ist und aus einem inneren Schutzbehälter für empfindliche Teile des Operationswerkzeugs und einer den Schutzbehälter aufnehmenden siegelfähigen Blisterverpackung besteht.

Vor diesem Hintergrund besteht die Aufgabe darin, eine Endoskopverpackung bereitzustellen, bei welcher das Risiko einer versehentlichen Kontamination eines im operativen Einsatz mit dem Patienten in Kontakt zu bringenden Abschnitts zu unterbinden oder zumindest zu reduzieren.

Diese Aufgabe wird bei einer erfindungsgemäßen Endoskopverpackung dadurch gelöst, dass das Aufnahmeteil und die Abdeckung derart konfiguriert oder aufeinander abgestimmt sind, dass beim ordnungsgemäßen Lösen der zweiteiligen Abdeckung von dem Aufnahmeteil zunächst nur die erste Kammer geöffnet wird; und erst anschließend, vorzugsweise nach dem vollständigen Öffnen der ersten Kammer, die zweite Kammer geöffnet wird; und, dass die zweiteilige Abdeckung eine erste Abdeckung aufweist, welche die erste Kammer verschließt, und eine zweite Abdeckung aufweist, welche die zweite Kammer verschließt, wobei sich die zweite Abdeckung erst nach Entfernen der ersten Abdeckung von dem Aufnahmeteil lösen lässt.

Vorteilhafte Ausgestaltungen der Erfindung sind Gegenstand der Unteransprüche.

Erfindungsgemäß weist das einstückige Aufnahmeteil zwei separate Kammern auf, wobei das Aufnahmeteil und die Abdeckung, mit welcher das Aufnahmeteil steril verschlossen bzw. verschließbar ist, derart konfiguriert sind, dass beim ordnungsgemäßen Lösen der Abdeckung von dem Aufnahmeteil die Kammern in einer vorbestimmten Reihenfolge nacheinander geöffnet werden.

Genauer gesagt dient eine erste Kammer zur Aufnahme eines ersten Endoskopabschnitts, der dafür bestimmt und ausgelegt ist, im operativen Einsatz nicht mit dem Patienten in Kontakt zu gelangen. Dabei kann es sich um den proximal angeordneten Handhabungs- oder Kopplungsabschnitt des Endoskops handeln. Die zweite Kammer dient zur Aufnahme eines zweiten Endoskopabschnitts, der dafür bestimmt und ausgelegt ist, im operativen Einsatz mit einem Patienten in Kontakt zu kommen. Dabei handelt es sich in der Regel um den Endoskopkopf samt dem gesamten oder großen Teil des Endoskopschlauchs.

Erfindungsgemäß gibt die Verpackung eine ganz bestimmte Anordnung der jeweiligen Endoskopabschnitte vor und erreicht dadurch, dass sich beim Lösen der Abdeckung bzw. Abziehfolie zunächst nur derjenige Abschnitt bzw. diejenige Kammer geöffnet wird, welche dem nicht mit dem Patienten in Berührung kommenden Abschnitt des Endoskops beinhaltet, und dass erst anschließend in einem zweiten Schritt derjenige Teil bzw. diejenige Kammer geöffnet wird, welche denjenigen Teil des Endoskops beinhaltet, der bis zum operativen Einsatz steril gehalten werden soll. Auf diese Weise lässt sich der proximale Abschnitt des Endoskops mit den Versorgungs- und Entsorgungsleitungen der Betriebsmaschine koppeln, ohne dabei Gefahr zu laufen, die noch unter Verschluss gehaltenen medialen und distalen Abschnitte des Endoskops zu kontaminieren. Somit kann durch eine geschickte Anordnung der entsprechenden Endoskopaufnahmeabschnitte in voneinander getrennten Kammern das Patientenrisiko wesentlich verringert bzw. die Anzahl versehentlich kontaminierter und somit nicht zum Einsatz kommender Endoskope verringert werden.

Die beiden Kammern können so konfektioniert sein, dass die jeweiligen Endoskopabschnitte auch nur in den für sie vorgesehenen Kammern Platz finden oder nur darin passen.

Gemäß einem Aspekt der Erfindung kann zwischen der ersten Kammer und der zweiten Kammer ein Steg ausgebildet sein, an welchem die Abdeckung mit dem Aufnahmeteil lösbar verbunden ist. Neben der örtlichen Trennung der beiden Kammern wird über den Steg ferner erreicht, dass die zweite Kammer selbst nach dem Öffnen der ersten Kammer umlaufend oder zumindest größtenteils hermetisch abgeschlossen bleibt. Auf diese Weise kann erreicht werden, dass die zweite Kammer selbst nach dem Öffnen der ersten Kammer vor Umwelteinflüssen weitestgehend geschützt bleibt.

Gemäß einem Aspekt kann der Steg eine Ausnehmung oder einen Durchbruch zur Aufnahme eines Zwischenstücks zwischen dem ersten Endoskopabschnitt und dem zweiten Endoskopabschnitt, vorzugsweise eines distalen Abschnitts des ersten Endoskopabschnitts, aufweisen. Auf diese Weise können der proximale erste Endoskopabschnitt und der distale zweite Endoskopabschnitt verbunden in der erfindungsgemäßen Endoskopverpackung ausgeliefert werden, so dass die Montage der beiden Abschnitte entfallen kann. Wenn die Breite der Ausnehmung auf die Breite des Zwischenstücks, bei dem es sich um einen proximalen Abschnitt des Endoskopschlauchs oder um einen distalen Abschnitt des Anschlussabschnitts handeln kann, beschränkt ist, ist die zweite Kammer nach dem Öffnen der ersten Kammer mit Ausnahme dieses kleinen Abschnitts der Ausnehmung im Steg komplett verschlossen.

Das Nacheinanderöffnen der ersten und zweiten Kammer in dieser vorbestimmten Reihenfolge kann auf verschiedene Weisen erreicht werden.

So kann beispielsweise die Abdeckung zweiteilig sein und eine erste Abdeckung aufweisen, welche die erste Kammer verschließt, und eine zweite Abdeckung, welche die zweite Abdeckung verschließt, wobei sich die zweite Abdeckung vorzugsweise erst nach Entfernung der ersten Abdeckung von dem Aufnahmeteil lösen lässt. Somit kann jede Kammer ihre eigene Abdeckung aufweisen. Wenn die erste Abdeckung und die zweite Abdeckung übereinander mit dem Aufnahmeteil, insbesondere mit dem Steg, verbunden sind, kann somit die zweite Abdeckung von dem Aufnahmeteil bzw. dem Steg erst nach dem Lösen der ersten Abdeckung entfernt werden. Auf diese Weise wird sehr einfach die Reihenfolge des Lösens der jeweiligen Abdeckungen oder Folien vorgegeben.

Alternativ kann die Abdeckung ebenfalls zweiteilig ausgebildet sein, wobei die erste Abdeckung sowohl die erste Kammer als auch die zweite Kammer verschließt und die unter der ersten Abdeckung angeordnete zweite Abdeckung lediglich die zweite Kammer verschließt. Auf diese Weise wird nach dem Lösen der ersten Abdeckung lediglich die erste Kammer geöffnet, während die zweite Abdeckung zum Vorschein kommt und nach wie vor die zweite Kammer verschließt. Zum Öffnen der zweiten Kammer ist dann die zweite Abdeckung von dem Aufnahmeteil zu lösen. Auf diese Weise kann mit größter Sicherheit verhindert werden, dass der Benutzer nicht versehentlich erst die zweite Abdeckung zu lösen beginnt, da diese durch die erste Abdeckung vollständig verdeckt wird.

Eine weitere Möglichkeit zum sequentiellen Öffnen der Kammern kann darin bestehen, die erste Kammer auf einer Seite oder Hälfte des Aufnahmeteils anzuordnen und die zweite Kammer auf einer anderen Seite oder Hälfte des Aufnahmeteils anzuordnen und beide Kammern mit einer einteiligen Abdeckung zu verschließen, wobei diese Abdeckung ausgelegt ist, in eine vorbestimmte Richtung von der Seite der ersten Kammer zur Seite der zweiten Kammer von dem Aufnahmeteil gelöst zu werden. Dies kann auf einfache Weise mittels einer (Abzieh-)Lasche erreicht werden, welche sich auf der einen bzw. auf der Seite der ersten Kammer befindet, so dass der Benutzer allein durch die Anordnung dieser Lasche veranlasst wird, die Abdeckung nur in eine bestimmte, nämlich in die gewünschte Richtung abzuziehen. Auf diese Weise kann die sequentielle Öffnung der Kammer auch nur mittels einer einteiligen Abdeckung erreicht werden.

Gemäß einem Aspekt der Erfindung kann an einem auf der Seite der zweiten Kammer gelegenen Randabschnitt des Aufnahmeteils eine Lasche zum Aufhängen der Verpackung vorgesehen sein. Wenn die Endoskopverpackung mit der Lasche aufgehängt wird, befindet sich die zweite Kammer im oberen Abschnitt und die erste Kammer im unteren Abschnitt der Verpackung. Somit kann durch Öffnen der ersten Kammer der proximale Abschnitt des Endoskops mit der Betriebsmaschine gekoppelt werden, während der mediale und distale Abschnitt des Endoskops in der nach wie vor verschlossenen zweiten Kammer gehalten wird. Dies erleichtert die Handhabung des Endoskops beim Anschließen an die Betriebsmaschine, da hierbei das Endoskop bzw. die Verpackung nicht gehalten werden muss und somit die Bedienperson die Hände zum Konnektieren der Ver- und Entsorgungsleitungen zur Verfügung hat.

Gemäß einem Aspekt der Erfindung kann in der ersten Kammer ein Aufnahmeabschnitt zur, vorzugsweise ortsfest fixierbaren, Aufnahme des ersten Endoskopabschnitts und ferner zumindest ein Aufnahmeabschnitt zur, vorzugsweise ortsfest fixierbaren, Aufnahme einer Komponente oder Peripherie, wie z. B. einem Kabel und/oder Stecker, für das Endoskop vorgesehen sein.

Zusätzlich oder alternativ kann in der zweiten Kammer ein Aufnahmeabschnitt zur, vorzugsweise ortsfest fixierbaren, Aufnahme des Endoskopschlauchs im spiralförmig aufgewickelten Zustand und ein Aufnahmeabschnitt zur, vorzugsweise ortsfest fixierbaren, Aufnahme des Endoskopkopfes, insbesondere eines Kameraabschnitts, vorgesehen sein.

Durch das Vorsehen bestimmter Aufnahmeabschnitte für bestimmte Endoskopabschnitte wird das Endoskop nicht nur in einer ganz bestimmten Lage in dem Aufnahmeteil angeordnet und gehalten, sondern auch das Einlegen und Verpacken des Endoskops nach der Sterilisation aufgrund konkreter Vorgaben erleichtert. Ferner können mit der Verpackung die für das jeweilige Endoskop benötigten Accessoires oder Peripheriekomponenten mitgeliefert werden. Durch die ortsfeste Positionierung und Fixierung wird auch sichergestellt, dass die jeweiligen Teile innerhalb der Verpackung nicht verrutschen und sich gegebenenfalls beim Transport gegenseitig beschädigen. Durch die Aufnahmemöglichkeit des Endoskopschlauchs im spiralförmig aufgewickelten Zustand wird nicht nur eine sehr platzsparende Aufbewahrung des Endoskopschlauchs ermöglicht, sondern auch eine Aufbewahrung, welche ein Knicken oder übermäßiges Biegen des Endoskopschlauchs und der darin vorgesehenen Leitungen und Kanäle unterbindet.

Zum Stützen des Endoskopschlauchs im aufgewickelten Zustand kann auch der Steg zwischen beiden Kammern zumindest auf der Seite der zweiten Kammer (kreis-)bogenförmig ausgebildet sein, um so eine (kreis-)bogenförmige Anlagefläche für den Endoskopschlauch zu bieten.

Um den Endoskopschlauch im spiralförmig aufgewickelten Zustand und/oder den Endoskopkopf nieder zu halten, kann die Verpackung ferner einen Deckel aufweisen, der in die zweite Kammer passgenau einbringbar und gegebenenfalls befestigbar ist. Auf diese Weise kann die Flächenlast, welche gegebenenfalls durch den zwanghaft aufgewickelten Endoskopschlauch auf die Abdeckung wirkt, über die vergrößerte Deckelfläche reduziert werden. Darüber hinaus werden durch die Deckelfläche etwaige Hohlräume verschlossen, wodurch die Abdeckung nicht von außen in diese Hohlräume eingedrückt und dadurch gegebenenfalls beschädigt werden kann.

Gemäß einem Aspekt der Erfindung kann der Abschnitt zur Aufnahme des ersten Endoskopabschnitts und der Abschnitt zu Aufnahme des Endoskopkopfs in einer Längsrichtung, vorzugsweise entlang einer Längskante des Aufnahmeteils, hintereinander angeordnet sein und so eine entsprechende Anordnung des ersten Endoskopabschnitts und des Endoskopkopfes ermöglichen. Auf diese Weise ergibt sich eine sehr platzsparende Anordnung der nicht flexiblen Abschnitte des Endoskops.

Gemäß einem Aspekt der Erfindung kann der Aufnahmeabschnitt für den Endoskopschlauch eine Vielzahl, vorzugsweise vier, von in Umfangsrichtung gleichmäßig verteilte, insbesondere bogenförmige, Halteelemente zur radialen Positionierung der jeweiligen Endoskopschlauchabschnitte aufweisen. Diese Halteelemente können anstatt an dem Aufnahmeabschnitt auch an dem Deckel vorgesehen sein. Durch diese Halteelemente wird der Endoskopschlauch ortsfest in der spiralförmig aufgewickelten Form radial nach innen durch die Halteelemente und radial nach außen durch Außenwandabschnitte des Aufnahmeteils und des Stegs in Position gehalten.

Gemäß einem Aspekt der Erfindung kann in der ersten Kammer eine Einlage mit einem zapfen- oder zylinderförmigen Vorsprung passgenau einbringbar sein, an welchem zumindest ein Kabel aufwickelbar ist. Durch passgenaue Form kann sichergestellt werden, dass die Einlage innerhalb der ersten Kammer nicht verrutscht. Mittels der Einlage wird auch erreicht, dass die Konfektionierung (Positionierung und Fixierung) der Kabel außerhalb der Verpackung bzw. des Aufnahmeteils vorgenommen werden kann und erst anschließend vorkonfektioniert in das Aufnahmeteil eingebracht werden kann. Dadurch wird der Verpackungsvorgang vereinfacht. Alternativ kann der zapfen- oder zylinderförmige Vorsprung auch am Boden der ersten Kammer des Aufnahmeteils ausgebildet sein.

Ein weiterer Aspekt der Erfindung betrifft ein Aufnahmeteil für eine Sterilverpackung nach einem der vorgehenden Aspekte mit einer ersten und einer zweiten Kammer und einem zwischen beiden Kammern angeordneten Steg, der die beiden Aufnahmeabschnitte voneinander trennt, wobei die beiden Kammern zusammen einen umlaufend geschlossenen Rand ausbilden und eine an dem Rand ausgebildete Fügefläche sowie eine am Steg ausgebildete Fügefläche in der gleichen Ebene liegen.

### Figurenbeschreibung

Die vorliegende Erfindung wird nachfolgend anhand eines bevorzugten Ausführungsbeispiels mit Bezugnahme auf die beigefügten Figuren näher erläutert. Es zeigen:
Fig. 1 zeigt eine perspektivische Ansicht einer Sterilverpackung gemäß einer bevorzugten Ausführungsform der Erfindung mit eingelegtem Endoskop;
Fig. 2 zeigt Draufsicht auf die Verpackung;
Fig. 3 zeigt die Schnittansicht III-III in der Fig. 2
Fig. 4 zeigt eine Seitenansicht der Verpackung;

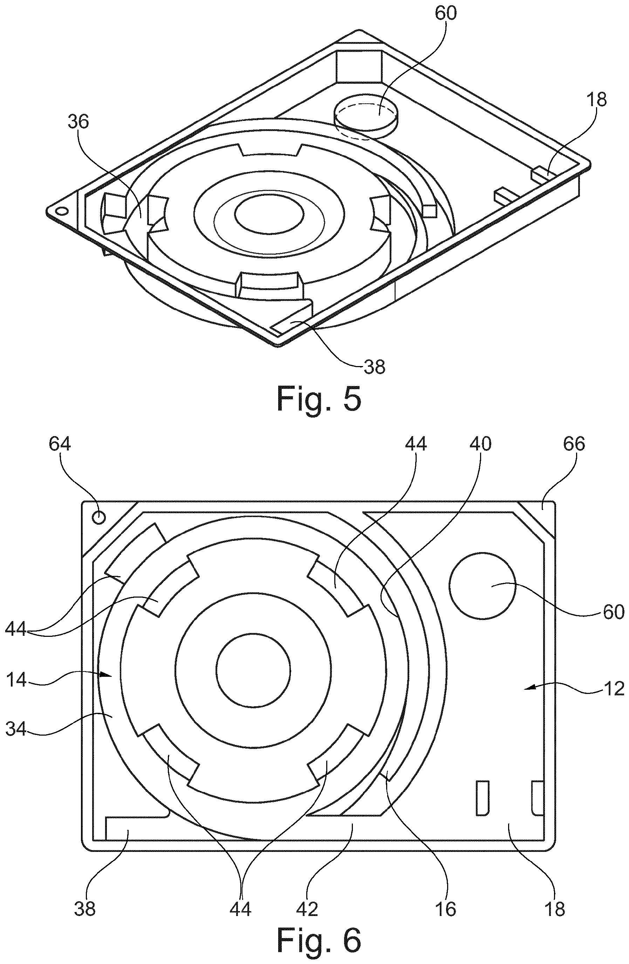

Fig. 5 zeigt eine perspektivische Ansicht des leeren Aufnahmeteils;
Fig. 6 zeigt Draufsicht auf das leere Aufnahmeteil;
Fig. 7A zeigt eine Seitenansicht eines Deckels der Verpackung;
Fig. 7B zeigt eine Unteransicht des Deckels;
Fig. 7C zeigt eine perspektivische Unteransicht des Deckels;
Fig. 8A zeigt eine perspektivische einer Einlage der Verpackung;
Fig. 8B zeigt eine Seitenansicht der Einlage;
Fig. 8C zeigt perspektivische Unteransicht der Einlage; und
Fig. 9 zeigt Fügeflächen zwischen Aufnahmeteil und Abdeckung.

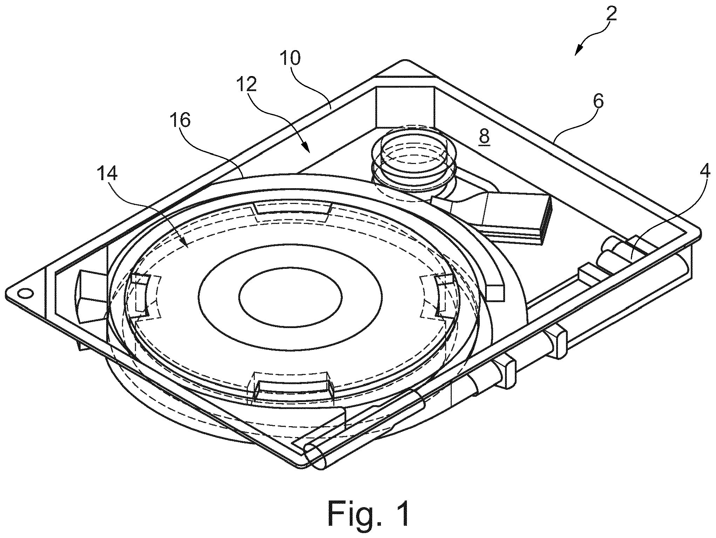

Die Fig. 1 zeigt eine perspektivische Ansicht einer erfindungsgemäßen Verpackung 2, welche zur sterilen Aufbewahrung eines Endoskops 4 geeignet ist. Die Verpackung 2 weist ein im Wesentlichen rechteckförmiges, schalenförmiges Aufnahmeteil 6 auf und eine Abdeckung 8 in Form einer dünnen Folie, welche an einen umlaufenden Rand 10 des Aufnahmeteils 6 angebracht und mit diesem lösbar (abziehbar) verbindbar ist, um das Innere des Aufnahmeteils 6 dicht bzw. steril zu verschließen. Anstelle einer Kunststofffolie kann auch anderes Material verwendet werden, solange dieses den Anforderungen genügt, das innerhalb des Aufnahmeteils 6 aufgenommene Endoskop dauerhaft steril zu halten. Das Aufnahmeteil 6 kann selbst ein Kunststofffolienformteil sein, dessen Kontur z.B. mittels Thermoformtechnik ausgebildet wird. Das Aufnahmeteil 6 weist im Wesentlichen zwei Kammern 12 und 14 auf, welche über einen dazwischen angeordneten Steg 16 räumlich voneinander getrennt werden.

Die erste Kammer 12 dient zur Aufnahme desjenigen Endoskopabschnitts und weiterer Komponenten, welche nicht unmittelbar mit einem Patienten in Kontakt kommen, und ist dementsprechend hinsichtlich Größe und Formgebung ausgelegt und angepasst. Die zweite Kammer 14 dagegen ist für die Aufnahme desjenigen Endoskopabschnitts vorgesehen, welcher im operativen Einsatz mit dem Patienten in Kontakt kommt, und ist ebenfalls dementsprechend hinsichtlich Größe und Formgebung ausgelegt und angepasst. Vor diesem Hintergrund sind die Reinheitsanforderungen hinsichtlich des zweiten Abschnitts deutlich höher als die des ersten Endoskopabschnitts.

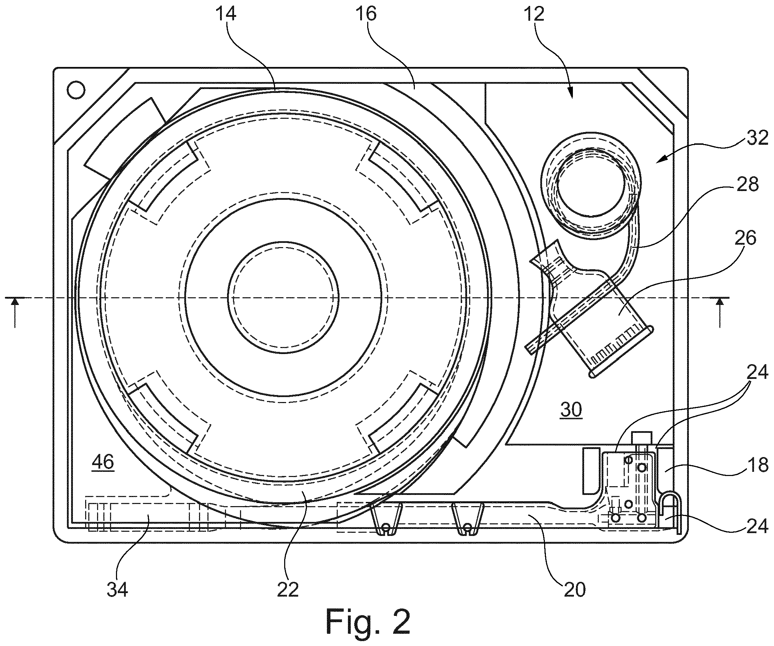

Die Fig. 2 zeigt eine Draufsicht auf die Verpackung 2 bzw. auf das Aufnahmeteil 6 und zeigt die erste Kammer 12 auf der rechten Seite und die zweite Kammer 14 auf der linken Seite. Die erste Kammer 12 weist zum einen einen Aufnahmeabschnitt 18 auf, der im Wesentlichen L-förmig ausgebildet ist und sich entlang einer Längskante des Aufnahmeteils 6 erstreckt. Dieser Aufnahmeabschnitt 18 dient zur Aufnahme eines im Wesentlichen L-förmigen Anschlusseinsatzes oder Kopplungsabschnitts 20, der am proximalen Ende eines Endoskopschlauchs 22 angeordnet ist. Dieser verfügt über einen nicht näher beschriebenen innenliegenden Arbeitskanal sowie diverse Arbeitskanalanschlüsse 24, welche mit entsprechenden Ver- und Entsorgungsleitungen von und zu einer nicht dargestellten Betriebsmaschine verbunden werden können. Bei diesem Anschlusseinsatz 20 handelt es sich um ein steifes Bauteil, während der daran anschließende Endoskopschlauch 22 flexibel ist und wie in der Fig. 2 gezeigt aufgewickelt werden kann.

In der ersten Kammer 12 ist ferner Platz für andere Peripheriegeräte und Accessoires, wie z. B. den in der Fig. 2 dargestellten Stecker 26 samt Kabel 28. Der Stecker 26 und das Kabel 28 sind mittels einer separaten Einlage 30, welche später näher beschrieben wird, in einen Aufnahmeabschnitt 32 in der ersten Kammer 12 einsetzbar.

Der Aufnahmeabschnitt 18 für den Endoskopanschluss 20 ist so ausgebildet, dass der Anschlusseinsatz 20, wenn dieser eingelegt ist, in seitlicher Richtung in seiner Position gehalten wird. Der Aufnahmeabschnitt 18 ist somit der Außenkontur des Anschlusseinsatzes 20 angepasst. Ferner kann durch die Ausbildung kleiner elastischer Rastnasen der Anschlusseinsatz 20 auch in Höhenrichtung in dem Aufnahmeabschnitt 18 gehalten werden.

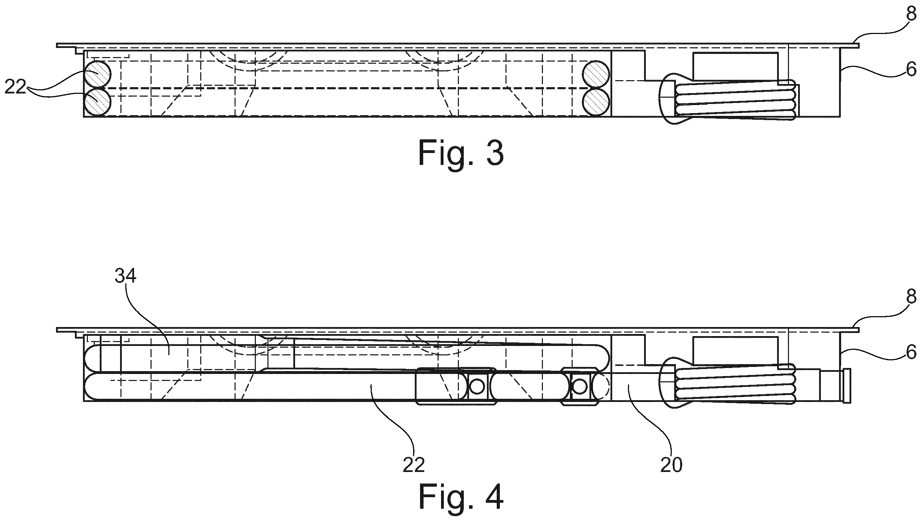

Der Endoskopschlauch 22 wird samt dem am distalen Ende des Endoskopschlauchs 22 angeordneten Endoskopkopf 34 in die zweite Kammer 14 eingesetzt, welcher sich in der Fig. 2 auf der linken Seite des Stegs 16 befindet. Der Endoskopschlauch 22 findet im aufgewickelten Zustand Platz in einem ringnutförmigen Aufnahmeabschnitt 36 und der zylindrische Endoskopkopf 34 in einem entsprechend dieser Kontur angepassten Aufnahmeabschnitt 38, der neben und längs derselben Längskante ausgebildet ist wie der Aufnahmeabschnitt 18 des Endoskopanschlusseinsatzes 20, so dass sich die Achsen des Anschlusseinsatzes 20 und des Endoskopkopfes 34 in die gleiche Richtung erstrecken. Der ringnutförmige Aufnahmeabschnitt 32 für den Endoskopschlauch 22 ist so dimensioniert, dass dieser zwei Wicklungen des Endoskopschlauchs 22 übereinander aufnehmen kann, wie dies in der Schnittansicht der Fig. 3 dargestellt ist. Aufgrund der Wicklung des Endoskopschlauchs übereinander, befindet sich der Aufnahmeabschnitt 38 für den Endoskopkopf 34 in der zweiten Kammer 14 um einen Endoskopschlauchdurchmesser höher in dem Aufnahmeteil 6 als der Anschlusseinsatz 20 in dem Aufnahmeabschnitt 18 in der ersten Kammer 12.

Der Steg 16, der die beiden Kammern 12 und 14 voneinander trennt, ist im Wesentliche bogenförmig ausgebildet und bietet somit auf der der zweiten Kammer 14 zugewandten Seite eine kreisbogenförmige Anlagefläche 40 für den Endoskopschlauch 22. Der Steg 16 trennt die beiden Kammern 12 und 14 nicht vollständig voneinander, sondern hat auf einer Seite randseitig einen Durchbruch 42 für den distalen Teil des Anschlusseinsatzes 20. Dieser Durchbruch 42 bildet die Schnittstelle zwischen der ersten Kammer 12 und der zweiten Kammer 14, um so das einteilige Endoskop 4 im Ganzen in dem Aufnahmeteil 6 platzieren zu können, mit den entsprechenden Abschnitten in den entsprechenden Kammern.

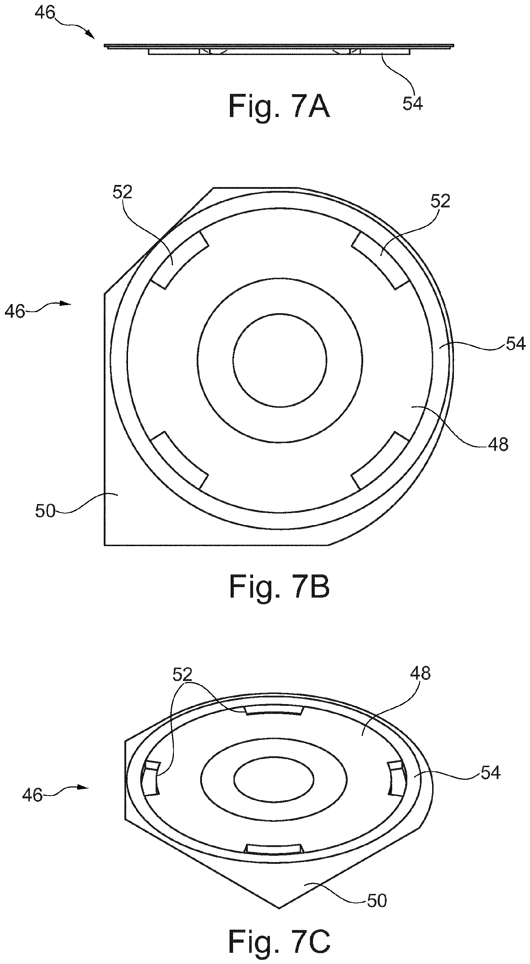

Zum leichteren Einlegen und Entnehmen des Endoskopschlauchs weist der ringnutförmige Aufnahmeabschnitt 36 für den Endoskopschlauch 22 in Umfangsrichtung verteilte radiale Eingriffe 44 auf. Um den in die ringnutförmige Aufnahme 36 eingelegten und aufgewickelten Endoskopschlauch 22 sowie den daran distal anschließenden Endoskopkopf 34 nieder zu halten, ist ein (separater) Deckel 46 (siehe Fig. 7A, 7B, 7C) vorgesehen, welcher der Kontur der zweiten Kammer 14 angepasst ist und die Kammer 14 von oben flächig verschließen kann. Dabei weist der flache Deckel 46 einen im Wesentlichen kreisförmigen Abschnitt 48 zum Niederhalten des Endoskopschlauchs und einen eckförmigen Abschnitt 50 zum Niederhalten des Endoskopkopfs 34 auf. Der Deckel 46 weist an der Unterseite bzw. auf der dem Endoskopschlauch 36 zugewandten Seite vier in Umfangsrichtung radial nach innen vorstehende Vorsprünge 52 auf, welche komplementär zu den Eingriffen 44 in der zweiten Kammer 14 ausgebildet sind und in diese eintauchen, wenn der Deckel 46 auf der zweiten Kammer 14 platziert wird. Ein ringförmiger Abschnitt 54 taucht beim Platzieren des Deckels 46 auf der Kammer 14 in die ringförmige Aufnahme 36 ein.

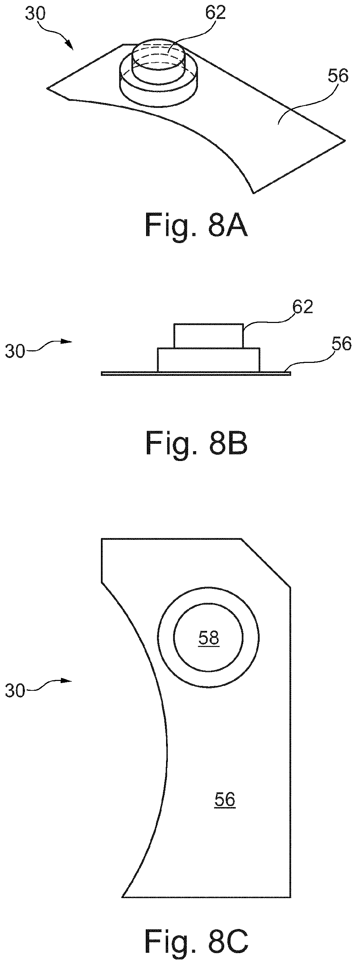

Die Fign. 8A, 8B und 8C zeigen verschiedene Ansichten der Einlage 30, welche in dem entsprechenden Aufnahmeabschnitt 32 der ersten Kammer 12 platziert werden kann. Diese Einlage 30 weist einen dünnlagigen, flächigen Bodenabschnitt 56 auf, dessen Außenkontur der Form der ersten Kammer 12 angepasst ist, so dass die Einlage 30 in dem Aufnahmeabschnitt 32 nicht seitlich hin- und herrutschen kann. Die Einlage 30 weist an ihrer Unterseite (siehe Fig. 8C) eine zylindrische Ausnehmung 58 auf, in welche ein zylindrischer Vorsprung 60, der am Boden des Aufnahmeabschnitts 32 bzw. des Aufnahmeteils 6 ausgebildet ist, passgenau eintaucht, wenn die Einlage 30 in der ersten Kammer 12 platziert wird. Die zylindrische Ausnehmung 58 bildet auf der Oberseite der Einlage 30 einen (gestuften) zylindrischen Vorsprung 62, an welchem das Kabel 28 aufgewickelt werden kann und im vorkonfektionierten Zustand samt Einlage 30 in den Aufnahmeabschnitt 32 in der ersten Kammer 12 eingesetzt werden kann.

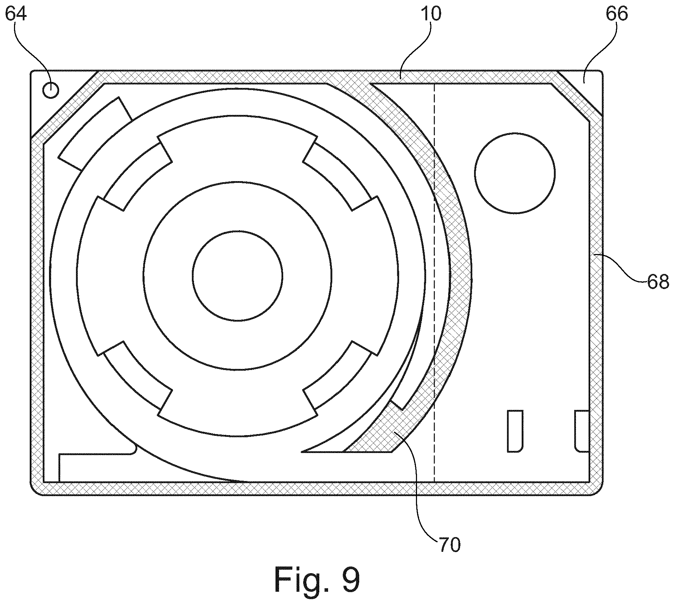

Das Aufnahmeteil 6 weist an einer Ecke (siehe Fig. 2 oder Fig. 9), und zwar auf der Seite der zweiten Kammer 14 und auf der zum Endoskopkopf 34 gegenüberliegenden Seite, eine Aufhängelasche 64 auf, an welcher die Verpackung 2 aufgehängt werden kann.

Auf der Seite der ersten Kammer 12 befindet sich an einer Ecke des Aufnahmeteils 6, und zwar auf der dem Anschlusseinsatz 20 gegenüberliegenden Seite bzw. auf der gleichen Seite wie die Lasche 64, eine Abziehlasche 66, an welcher die Abdeckung 8 nicht mit dem Aufnahmeteil 6 verbunden ist und von dort aus gegriffen und entfernt bzw. von dem Rand 10 abgezogen werden kann. Von daher wird beim Öffnen der Abdeckung 8 - ausgehend von der Ecke der Abziehlasche 66 - zunächst die erste Kammer 12 auf der Seite der Abziehlasche 66 und erst anschließend die zweite Kammer 14 geöffnet. Auf diese Weise kann zunächst die Abdeckung 8 nur so weit geöffnet werden, dass der in der ersten Kammer 12 befindliche Anschlusseinsatz 20 über die entsprechenden Arbeitskanalanschlüsse 24 mit den Ver- und Entsorgungsleitungen der Betriebsmaschine gekoppelt werden kann, ohne dabei die zweite Kammer 14 öffnen zu müssen und somit eine Kontamination des steril zu haltenden Endoskopschlauchs 22 samt Endoskopkopf 34 zu riskieren.

Da die Abdeckung 8 ferner im Bereich des Stegs 16 mit dem Aufnahmeteil 6 dicht verbunden ist oder sein kann, wird die Gefahr einer Kontamination der zweiten Kammer 14 nach dem Öffnen der ersten Kammer 12 minimiert.

Die Erfindung wurde anhand einer bevorzugten Ausführungsform beschrieben, ist jedoch auf diese nicht beschränkt. Erfindungsgemäss werden anstelle einer einzigen Abdeckung 8 zwei übereinander angeordnete und sich teilweise überlappende Abdeckungen bzw. Folien verwendet. Beispielsweise kann die erste Abdeckung beide Kammern 12 und 14 und die zweite Abdeckung lediglich die zweite Kammer 14 verschließen, so dass nach dem Abziehen der ersten Abdeckung oder Folie die erste Kammer 12 freigegeben wird, während die zweite Kammer 14 durch die zweite Folie verschlossen bleibt.

Des Weiteren kann das erfindungsgemäße Verpackungsprinzip auch auf Endoskope mit starren Schäften angewandt werden.

Darüber hinaus ist das erfindungsgemäße Verpackungsprinzip grundsätzlich für alle medizinischen Instrumente und Gerätschaften anwendbar, welche einen Abschnitt aufweisen, welcher mit dem Patienten unmittelbar in Kontakt gelangt, und einen anderen Abschnitt, der lediglich vom Chirurgen oder Benutzer betätigt wird. Somit könnte das erfindungsgemäße Prinzip auch bei Kathetern und ähnliches eingesetzt werden, solange derjenige Abschnitt, der mit dem Patienten tatsächlich in Kontakt kommt, in der zweiten Kammer platziert wird und der Teil, der mit dem Patienten nicht in Kontakt kommt, in der ersten Kammer angeordnet wird.

Zusammenfassend ergibt sich somit eine Verpackung für medizinische Instrumente und Gerätschaften mit einem Aufnahmeteil zur Aufnahme des Instruments und einer zumindest mit dem Aufnahmeteil randseitig verbundenen zweiteiligen Abdeckung zum sterilen Verschließen des Aufnahmeteils, wobei das Aufnahmeteil aufweist: eine erste Kammer zur Aufnahme eines ersten Instrumentenabschnitts, der im operativen Einsatz nicht mit dem Patienten in Kontakt gelangt, und eine zweite Kammer zur Aufnahme eines zweiten Instrumentenabschnitts, der im operativen Einsatz mit einem Patienten in Kontakt kommt, wobei das Aufnahmeteil und die Abdeckung derart konfiguriert oder aufeinander abgestimmt sind, dass beim ordnungsgemäßen Lösen der Abdeckung von dem Aufnahmeteil zunächst nur die erste Kammer geöffnet wird und erst anschließend die zweite Kammer geöffnet wird.

### Bezugszeichenliste

- 2: Verpackung
- 4: Endoskop
- 6: Aufnahmeteil
- 8: Abdeckung
- 10: Rand
- 12: Erste Kammer
- 14: Zweite Kammer
- 16: Steg
- 18: Aufnahmeabschnitt (Anschlusseinsatz)
- 20: Anschlusseinsatz bzw. Kopplungsabschnitt
- 22: Endoskopschlauch
- 24: Arbeitskanal bzw. Arbeitskanalanschlüsse
- 26: Stecker
- 28: Kabel
- 30: Einlage
- 32: Aufnahmeabschnitt (Einlage)
- 34: Endoskopkopf
- 36: Aufnahmeabschnitt (Endoskopschlauch)
- 38: Aufnahmeabschnitt (Endoskopkopf)
- 40: Anlagefläche
- 42: Durchbruch
- 44: Eingriff
- 46: Deckel
- 48: Kreisabschnitt
- 50: Eckabschnitt
- 52: Radialvorsprung
- 54: Ringabschnitt
- 56: Bodenabschnitt
- 58: Ausnehmung
- 60: Vorsprung
- 62: Vorsprung
- 64: Aufhängelasche
- 66: Abziehlasche

## Patentansprüche

1. Endoskopverpackung (2) mit einem einstückig ausgebildeten Aufnahmeteil (6) zur Aufnahme eines Endoskops (4) und zumindest einer mit dem Aufnahmeteil (6) zumindest randseitig lösbar verbundenen zweiteiligen Abdeckung (8) zum sterilen Verschließen des Aufnahmeteils (6),
wobei das Aufnahmeteil (6) aufweist:
eine erste Kammer (12) zur Aufnahme eines ersten Endoskopabschnitts (20), der dafür bestimmt und ausgelegt ist, im operativen Einsatz nicht mit dem Patienten in Kontakt zu gelangen, und
eine zweite Kammer (14) zur Aufnahme eines zweiten Endoskopabschnitts (22, 34), der dafür bestimmt und ausgelegt ist, im operativen Einsatz mit einem Patienten in Kontakt zu kommen; und
wobei das Aufnahmeteil (6) und die zweiteilige Abdeckung (8) derart konfiguriert oder aufeinander abgestimmt sind, dass beim ordnungsgemäßen Lösen der Abdeckung (8) von dem Aufnahmeteil (6) zunächst nur die erste Kammer (12) geöffnet wird; und erst anschließend die zweite Kammer (14) geöffnet wird;
und die Abdeckung (8) eine erste Abdeckung aufweist, welche die erste Kammer (12) verschließt, und eine zweite Abdeckung aufweist, welche die zweite Kammer (14) verschließt, wobei sich die zweite Abdeckung erst nach Entfernen der ersten Abdeckung von dem Aufnahmeteil (6) lösen lässt.

2. Endoskopverpackung (2) nach Anspruch 1, **dadurch gekennzeichnet, dass** zwischen der ersten Kammer (12) und der zweiten Kammer (14) ein Steg (16) ausgebildet ist, an welchem vorzugsweise die Abdeckung (8) mit dem Aufnahmeteil (6) lösbar verbunden ist.

3. Endoskopverpackung (2) nach Anspruch 2, **dadurch gekennzeichnet, dass** der Steg (16) zumindest teilweise bogenförmig ausgebildet ist und auf der Seite der zweiten Kammer (14) eine bogenförmige Anlagefläche (40) für den Endoskopschlauch (22) aufweist und/oder der Steg (16) eine Ausnehmung (42) zur Aufnahme eines Zwischenstücks zwischen dem ersten Endoskopabschnitt (20) und dem zweiten Endoskopabschnitt (22, 34) aufweist.

4. Endoskopverpackung (2) nach einem der vorhergehenden Ansprüche, **dadurch gekennzeichnet, dass** die erste Abdeckung die erste Kammer (12) und die zweite Kammer (14) verschließt, und die unter der ersten Abdeckung angeordnete zweite Abdeckung lediglich die zweite Kammer (14) verschließt.

5. Endoskopverpackung (2) nach einem der Ansprüche 1 bis 3, **dadurch gekennzeichnet, dass** die erste Kammer (12) auf einer Seite oder Hälfte des Aufnahmeteils (6) angeordnet ist, die zweite Kammer (14) auf einer anderen Seite oder Hälfte des Aufnahmeteils (6) angeordnet ist, und die Abdeckung derart ausgelegt ist, insbesondere auf der Seite der ersten Kammer (12) eine Lasche (66) aufweist, um in eine vorbestimmte Richtung von der eine Seite zu der anderen Seite von dem Aufnahmeteil (6) gelöst zu werden.

6. Endoskopverpackung (2) nach einem der vorhergehenden Ansprüche, **dadurch gekennzeichnet, dass** an einem auf der Seite der zweiten Kammer (14) gelegener Randabschnitt (10) des Aufnahmeteils (6) eine Lasche (64) zum Aufhängen der Verpackung (2) vorgesehen ist.

7. Endoskopverpackung (2) nach einem der vorhergehenden Ansprüche, **dadurch gekennzeichnet, dass** in der ersten Kammer (12) ein Aufnahmeabschnitt (18) zur, vorzugsweise ortsfest fixierbaren oder passgenauen, Aufnahme des ersten Endoskopabschnitts (20) und ferner zumindest einen Aufnahmeabschnitt (32) zur, vorzugsweise ortsfest fixierbaren oder passgenauen, Aufnahme einer Komponente oder Peripherie, wie Kabel (28) und/oder Stecker (26), für das Endoskop (4) vorgesehen ist; und/oder
in der zweiten Kammer (14) ein Aufnahmeabschnitt (36) zur, vorzugsweise ortsfest fixierbaren oder passgenauen, Aufnahme des Endoskopschlauchs (22) im spiralförmig aufgewickelten Zustand und ein Aufnahmeabschnitt (38) zur, vorzugsweise ortsfest fixierbaren oder passgenauen, Aufnahme des Endoskopkopfes (34), insbesondere eines Kameraabschnitts, vorgesehen ist.

8. Endoskopverpackung nach einem der vorhergehenden Ansprüche, **dadurch gekennzeichnet, dass** der Abschnitt (18) zur Aufnahme des ersten Endoskopabschnitts (20) und der Abschnitt (38) zur Aufnahme des Endoskopkopfes (34) eine Positionierung des ersten Endoskopabschnitts (20) und des Endoskopkopfes (34) hintereinander in einer Längsrichtung, vorzugsweise entlang einer Längskante des Aufnahmeteils (6), ermöglichen.

9. Endoskopverpackung nach Anspruch 7 oder 8, ferner **gekennzeichnet durch**:
einen separaten Deckel (46), der in oder auf die zweite Kammer (14) passgenau an- oder einbringbar ist und derart konfiguriert ist, den Endoskopschlauch (22) und/oder den Endoskopkopf (34) niederzuhalten; und/oder
eine in die erste Kammer (12) passgenau einbringbare Einlage (30) mit einem zapfen- oder zylinderförmigen Vorsprung (62), an welchem zumindest ein Kabel (28) aufwickelbar ist.

10. Endoskopverpackung nach Anspruch 2, **dadurch gekennzeichnet, dass** die beiden Kammern (12, 14) zusammen einen umlaufenden geschlossenen Rand (10) ausbilden und eine an dem Rand (10) ausgebildete Fügefläche (68) und eine am Steg ausgebildete Fügefläche (70) in der gleichen Ebene liegen.

## Claims

1. Endoscope packaging (2) comprising a one-piece receiving part (6) for receiving an endoscope (4) and at least one two-part cover (8) releasably connected to the receiving part (6) at least on the edge for the sterile closing of the receiving part (6),
wherein the receiving part (6) comprises:
a first chamber (12) for receiving a first endoscope section (20), which is intended and designed so as not to come into contact with the patient in operative use, and
a second chamber (14) for receiving a second endoscope section (22, 34), which is intended and designed so as to come into contact with a patient in operative use, and
wherein the receiving part (6) and the two-part cover (8) are configured or coordinated with one another such that during the correct removal of cover (8) from the receiving part (6) firstly only the first chamber (12) is opened, and only then is the second chamber (14) opened,
and the cover (8) comprises a first cover, which closes the first chamber (12) and comprises a second cover, which closes the second chamber (14), wherein the second cover can only be released from the receiving part (6) after the removal of the first cover.

2. Endoscope packaging (2) according to claim 1, **characterised in that** between the first chamber (12) and the second chamber (14) a web (16) is formed, on which preferably the cover (8) is connected releasably to the receiving part (6).

3. Endoscope packaging (2) according to claim 2, **characterised in that** the web (16) is designed to be at least partly arched and on the side of the second chamber (14) comprises an arched bearing surface (40) for the endoscope tube (22) and/or the web (16) has a recess (42) for receiving an intermediate piece between the first endoscope section (20) and the second endoscope section (22, 34).

4. Endoscope packaging (2) according to any of the preceding claims, **characterised in that** the first cover closes the first chamber (12) and the second chamber (14) and the second cover arranged underneath the first cover only closes the second chamber (14).

5. Endoscope packaging (2) according to any of claims 1 to 3, **characterised in that** the first chamber (12) is arranged on one side or half of the receiving part (6), the second chamber (14) is arranged on another side or half of the receiving part (6), and the cover is designed such that, in particular on the side of the first chamber (12), it has a tab (66) in order to be removed from the receiving part (6) in a predetermined direction from one side to the other side.

6. Endoscope packaging (2) according to any of the preceding claims, **characterised in that** on an edge section (10) of the receiving part (6) on the side of the second chamber (14) a tab (64) is provided for hanging up the packaging (2).

7. Endoscope packaging (2) according to any of the preceding claims, **characterised in that** in the first chamber (12) a receiving section (18) is provided for receiving the first endoscope section (20), preferably so as to be fixed in place or to fit exactly, and further at least one receiving section (32) is provided for receiving a component or a periphery, preferably so as to be fixed in place or to fit exactly, such as a cable (28) and/or plug (26) for the endoscope (4), and/or
in the second chamber (14) a receiving section (36) is provided for receiving the endoscope tube (22), preferably so as to be fixed in place or to fit exactly, in the spiral wound state and a receiving section (38) is provided for receiving the endoscope head (34), preferably so as to be fixed in place or to fit exactly, in particular a camera section.

8. Endoscope packaging according to any of the preceding claims, **characterised in that** the section (18) for receiving the first endoscope section (20) and the section (38) for receiving the endoscope head (34) enable the positioning of the first endoscope section (20) and the endoscope head (34) behind one another in longitudinal direction, preferably along a longitudinal edge of the receiving part (6).

9. Endoscope packaging according to claim 7 or 8, further **characterised by**:
a separate cover (46), which can be attached or inserted to fit exactly into or onto the second chamber (14) and is configured so as to hold down the endoscope tube (22) and/or endoscope head (34), and/or
an insert (30) to be inserted to fit exactly into the first chamber (12) with a stud-like or cylindrical projection (62), on which at least one cable (28) can be wound.

10. Endoscope packaging according to claim 2, **characterised in that** the two chambers (12, 14) together form a peripheral closed edge (10) and a joining surface (68) formed on the edge (10) and a joining surface (70) formed on the web are in the same plane.

## Revendications

1. Conditionnement pour endoscope (2) avec une partie de réception (6) réalisée d'un seul tenant pour la réception d'un endoscope (4) et au moins un recouvrement à deux parties (8) reliées au moins côté bord de manière amovible à la partie de réception (6) pour la fermeture stérile de la partie de réception (6),
dans lequel la partie de réception (6) présente :
un premier compartiment (12) pour la réception d'une première section d'endoscope (20), qui est destinée et conçue pour ne pas entrer en contact avec le patient en utilisation opérationnelle, et
un deuxième compartiment (14) pour la réception d'une deuxième section d'endoscope (22, 34), qui est destinée et conçue pour entrer en contact avec un patient en utilisation opérationnelle ; et
dans lequel la partie de réception (6) et le recouvrement à deux parties (8) sont configurés ou adaptés l'un à l'autre de sorte que lors du détachement correct du recouvrement (8) de la partie de réception (6), seul le premier compartiment (12) est d'abord ouvert ; et ensuite seulement le deuxième compartiment (14) est ouvert ;
et le recouvrement (8) présente un premier recouvrement, qui ferme le premier compartiment (12), et présente un deuxième recouvrement, qui ferme le deuxième compartiment (14), dans lequel le deuxième recouvrement ne peut être détaché de la partie de réception (6) qu'après le retrait du premier recouvrement.

2. Conditionnement pour endoscope (2) selon la revendication 1, **caractérisé en ce qu'**une nervure (16), au niveau de laquelle de préférence le recouvrement (8) est relié de manière amovible à la partie de réception (6), est réalisée entre le premier compartiment (12) et le deuxième compartiment (14).

3. Conditionnement pour endoscope (2) selon la revendication 2, **caractérisé en ce que** la nervure (16) est réalisée au moins en partie en forme d'arc et présente sur le côté du deuxième compartiment (14) une surface d'appui en forme d'arc (40) pour le tuyau d'endoscope (22) et/ou la nervure (16) présente un évidement (42) pour la réception d'une pièce intermédiaire entre la première section d'endoscope (20) et la deuxième section d'endoscope (22, 34).

4. Conditionnement pour endoscope (2) selon l'une quelconque des revendications précédentes, **caractérisé en ce que** le premier recouvrement ferme le premier compartiment (12) et le deuxième compartiment (14), et le deuxième recouvrement agencé sous le premier recouvrement ferme seulement le deuxième compartiment (14).

5. Conditionnement pour endoscope (2) selon l'une quelconque des revendications 1 à 3, **caractérisé en ce que** le premier compartiment (12) est agencé sur un côté ou moitié de la partie de réception (6), le deuxième compartiment (14) est agencé sur un autre côté ou moitié de la partie de réception (6), et le recouvrement est conçu de sorte à, présente en particulier sur le côté du premier compartiment (12) une patte (66), être desserré dans une direction prédéterminée de l'un côté vers l'autre côté de la partie de réception (6).

6. Conditionnement pour endoscope (2) selon l'une quelconque des revendications précédentes, **caractérisé en ce qu'**une patte (64) est prévue pour l'accrochage du conditionnement (2) au niveau d'une section de bord (10) de la partie de réception (6) située sur le côté du deuxième compartiment (14).

7. Conditionnement pour endoscope (2) selon l'une quelconque des revendications précédentes, **caractérisé en ce que** dans le premier compartiment (12) est prévue une section de réception (18) pour la réception, de préférence fixable de manière stationnaire ou en ajustement serré, de la première section d'endoscope (20) et en outre au moins une section de réception (32) pour la réception, de préférence fixable de manière stationnaire ou en ajustement serré, d'un composant ou périphérique, tel que câble (28) et/ou fiche (26), pour l'endoscope (4) ; et/ou
dans le deuxième compartiment (14) est prévue une section de réception (36) pour la réception, de préférence fixable de manière stationnaire ou en ajustement serré, du tuyau d'endoscope (22) à l'état enroulé en forme de spirale et une section de réception (38) pour la réception, de préférence fixable de manière stationnaire ou en ajustement serré, de la tête d'endoscope (34), en particulier d'une section de caméra.

8. Conditionnement pour endoscope selon l'une quelconque des revendications précédentes, **caractérisé en ce que** la section (18) pour la réception de la première section d'endoscope (20) et la section (38) pour la réception de la tête d'endoscope (34) permettent un positionnement de la première section d'endoscope (20) et de la tête d'endoscope (34) l'une derrière l'autre dans une direction longitudinale, en particulier le long d'une arête longitudinale de la partie de réception (6).

9. Conditionnement pour endoscope selon la revendication 7 ou 8, **caractérisé en outre par** :
un couvercle séparé (46), qui peut être monté ou introduit en ajustement serré dans ou sur le deuxième compartiment (14) et est configuré de sorte à maintenir en bas le tuyau d'endoscope (22) et/ou la tête d'endoscope (34); et/ou
un insert (30) pouvant être introduit en ajustement serré dans le premier compartiment (12) avec une saillie en forme de broche ou de cylindre (62), sur laquelle au moins un câble (28) peut être enroulé.

10. Conditionnement pour endoscope selon la revendication 2, **caractérisé en ce que** les deux compartiments (12, 14) forment ensemble un bord fermé circonférentiel (10) et une surface de jonction (68) réalisée au niveau du bord (10) et une surface de jonction (70) réalisée au niveau de la nervure se trouvent dans le même plan.
